# EUROPEAN PATENT APPLICATION

(11) **EP 2 040 187 A1**
(43) Date of publication of application: **25.03.2009**
(21) Application number: 08001627.2
(22) Date of filing: 29.01.2008
(51) Int. Cl.: G06F 19/00, C12Q 1/68

(54) **Gene information processing apparatus and gene information display apparatus**

(30) Priority: 29.03.2007 JP 2007087642
(71) Applicant: HITACHI SOFTWARE ENGINEERING CO., LTD., Shinagawa-ku 140-0002 Tokyo (JP)
(72) Inventor: Matsumoto, Toshiko, Tokyo 140-0002 (JP); Nakashige, Ryo, Tokyo 140-0002 (JP)
(74) Representative: Liesegang, Eva

(57) **Abstract**

For automatic genotype determination based on microsatellites, when a +A peak occurrence pattern largely varies due to excessively intense fluorescent signal, incapability of separating peaks completely, and incomplete experimental conditions, a decreased precision in noise removal and time requirement for visual check and correction become bottlenecks in the analysis. On the basis of data about alleles which have been reported to be observed in a marker, it is investigated whether or not a sample is suitable to be used for examining a +A peak occurrence pattern, which peak can be a starting point for examining the +A peak occurrence pattern, and whether the reported allele is a true peak or the +A peak thereof. According to the obtained data, the +A peak occurrence pattern is estimated. In this process, it is possible to perform an accurate estimation, and therefore, a precision for noise removal in automatic genotype discrimination can be improved.

SEQUENCE LISTING

<110> Hitachi Software Engineering Co., Ltd.

<120> GENE INFORMATION PROCESSING APPARATUS AND GENE
      INFORMATION DISPLAY APPARATUS

<130> PH-3331

<150> JP2007-087642
<151> 2007-03-30

<160> 12

<170> PatentIn Ver. 2.1

<210> 1
<211> 10
<212> DNA
<213> Artificial Sequence

<220>
<223> Description of Artificial
      Sequence:Synthetic DNA

<400> 1
acacacacac                                                        10


<210> 2
<211> 14
<212> DNA
<213> Artificial Sequence

<220>
<223> Description of Artificial
      Sequence:Synthetic DNA

<400> 2
acacacacac acac                                                   14

<210> 3
<211> 12
<212> DNA
<213> Artificial Sequence

<220>
<223> Description of Artificial
      Sequence:Synthetic DNA

<400> 3
acacacacac ac                                                          12


<210> 4
<211> 12
<212> DNA
<213> Artificial Sequence

<220>
<223> Description of Artificial
      Sequence:Synthetic DNA

<400> 4
acacacacac ac                                                          12


<210> 5
<211> 66
<212> DNA
<213> Artificial Sequence

<220>
<223> Description of Artificial
      Sequence:Synthetic DNA

<400> 5
ataatgatgg aaataattca tgtcaacaca cacacacaca cacacacact ccgacaatgt 60
ggagag                                                                 66


<210> 6
<211> 58

<212> DNA
<213> Artificial Sequence

<220>
<223> Description of Artificial
      Sequence:Synthetic DNA

<400> 6
ataatgatgg aaataattca tgtcaacaca cacacacaca ctccgacaat gtggagag   58


<210> 7
<211> 132
<212> DNA
<213> Artificial Sequence

<220>
<223> Description of Artificial
      Sequence:Synthetic DNA

<400> 7
ataatgatgg aaataattca tgtcaacaca cacacacaca cacacacact ccgacaatgt 60
ggagagataa tgatggaaat aattcatgtc aacacacaca cacacacaca cacactccga 120
caatgtggag ag                                                     132


<210> 8
<211> 116
<212> DNA
<213> Artificial Sequence

<220>
<223> Description of Artificial
      Sequence:Synthetic DNA

<400> 8
ataatgatgg aaataattca tgtcaacaca cacacacaca ctccgacaat gtggagagat 60
aatgatggaa ataattcatg tcaacacaca cacacacact ccgacaatgt ggagag     116


<210> 9
<211> 66

<212> DNA
<213> Artificial Sequence

<220>
<223> Description of Artificial
      Sequence:Synthetic DNA

<400> 9
ataatgatgg aaataattca tgtcaacaca cacacacaca cacacacact ccgacaatgt 60
ggagag                                                            66


<210> 10
<211> 199
<212> DNA
<213> Artificial Sequence

<220>
<223> Description of Artificial
      Sequence:Synthetic DNA

<400> 10
ataatgatgg aaataattca tgtcaacaca cacacacaca cacacacact ccgacaatgt 60
ggagagataa tgatggaaat aattcatgtc aacacacaca cacacacaca cacactccga 120
caatgtggag agataatgat ggaaataatt catgtcaaca cacacacaca cacacacaca 180
ctccgacaat gtggagaga                                              199


<210> 11
<211> 129
<212> DNA
<213> Artificial Sequence

<220>
<223> Description of Artificial
      Sequence:Synthetic DNA

<400> 11
ataatgatgg aaataattca tgtcaacaca cacacacaca cacacactcc gacaatgtgg 60
agagataatg atggaaataa ttcatgtcaa cacacacaca cacacacaca ctccgacaat 120
gtggagaga                                                         129

<210> 12
<211> 137
<212> DNA
<213> Artificial Sequence

<220>
<223> Description of Artificial
      Sequence:Synthetic DNA

<400> 12
ataatgatgg aaataattca tgtcaacaca cacacacaca cacacacaca ctccgacaat 60
gtggagagat aatgatggaa ataattcatg tcaacacaca cacacacaca cacacacact 120
ccgacaatgt ggagaga                                                137

## Description

### BACKGROUND OF THE INVENTION

### 1. Field of the Invention

The present invention relates to a gene information processing apparatus and a gene information display apparatus and, in particular, to a gene information apparatus and a gene information display apparatus which are used for analysis of a partial genomic sequence fragment where a polymorphism is observed among individuals. The invention especially directed at performing processing for discriminating between a signal of a target gene to be analyzed and a noise signal, when a DNA fragment containing the target gene is extracted and detected by using PCR technique and electrophoresis.

### 2. Description of the Related Art

### Background Art

Since the decoding of the human genome was completed, various studies on functional analysis of genes have been actively conducted. Among such studies, an automation technique for genotype determination providing a foundation for exploring genes involved in phenotypes, such as whether or not being affected by a certain disease, degree of drug effect, and whether or not having a side effect of a drug, has especially been receiving great attention. In order to improve accuracy in genotype determination process, it is preferable that a skilled person analyze each data visually and assemble the results of the analysis. Such a human-intensive analysis, however, has not been and will not be performed, since it is necessary to conduct analyses for a large amount of data. It is practical to employ an automated analysis using a computer, and, moreover, utilization of such an analysis can reduce variations in results caused by human error. In order to employ such an automated analysis, it is important to consider how to construct an algorithm for a computer automatic analysis, and how to obtain efficiently a highly precise result by, for example, discriminating a peak from a noise peak automatically.

The basics of a computer analysis will be explained in the following section.

### Microsatellite

Genomes of organisms of the same kind generally have approximately similar base sequences, but also contain different base sequences in some locations. For example, at a certain gene locus, an individual has A, while another has T. Having such a polymorphism in a single base in a genome among individuals is called an SNP (Single Nucleotide Polymorphism).

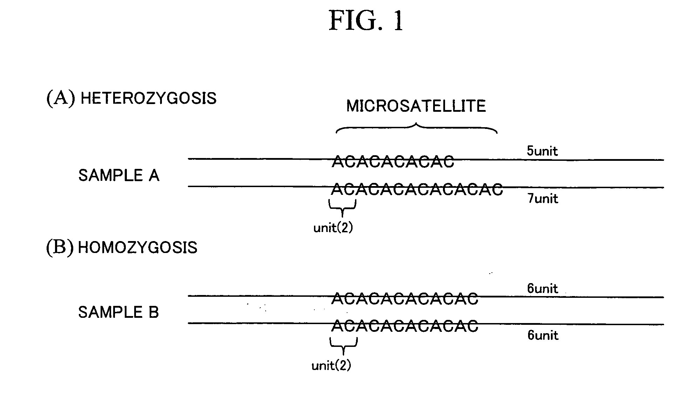

Meanwhile, a genome of an organism contains a large number of locations (more than several tens of thousands of locations) in which a few to several tens of repetitions of a relatively short sequence pattern containing 2 to 6 bases appear. Such a distinctive sequence pattern is called a microsatellite. Examples of microsatellites appearing in a genome are shown in Fig. 1. A base repeating unit in a microsatellite is called a "unit," and the number of bases in a unit is called "unit length." For example, in the microsatellite ACACACAC... (registered under a name "D20S910" in the public DNA database UniSTS at NIH of the United States) shown in Figs. 1 (A) and (B), the unit is "AC" and the unit length is 2 bases. Even if a unit and the unit length are the same between microsatellites, the number of repetitions may vary in some cases, as shown in Figs. 1(A) and (B).

As described above, since a SNP and microsatellite can vary among individuals, it is easy to distinguish a genome sequence of an individual from that of another by looking at SNP and microsatellite sequence portions in the genomes, and also easy to detect such sequence portions experimentally. For some organism species, approximate locations of SNPs and microsatellites in a genome are known; thus, such SNPs and microsatellites can be used as indicators for the location in a genome. For having such a property, SNPs and microsatellites are called DNA markers. A microsatellite, in particular, includes multiple bases, and therefore contains a larger amount of information than a SNP; thus, microsatellites have been frequently used as DNA markers.

As shown in Fig. 1, a large number of organism individuals include a pair of genomes (homologous chromosomes) derived from female and male gametes. Genes at corresponding sites on a pair of genomes are called alleles, and the combination of the alleles is called a genotype. As described above, base sequences in SNPs and microsatellites in a genome can vary among individuals, and, in general, a single SNP has 2 or 3 alleles, and a single microsatellite has several to more than 20 kinds of alleles.

In the example shown in Fig. 1(A), the sample A has five and seven repetitions of a unit "AC," while, in the example shown in Fig. 1(B), the sample B has a pair of six repetitions of a unit "AC". As shown in these examples, having two alleles of different kinds, such as the sample A, is called heterozygosis, and having two alleles of the same kind, such as the sample B, is called homozygosis.

### Experiments by using PCR technique and electrophoresis

When a microsatellite is used as a DNA marker, a PCR (Polymerase Chain Reaction) technology and electrophoresis are performed for detecting a DNA fragment containing the microsatellite in a genome. In the PCR technique, a pair of base sequences, called primer sequences, is designated at both ends of a target microsatellite, and only the sequence fragment between the primer sequences can be repeatedly replicated by using the primer sequences during the reaction. As a result, a certain amount of the DNA fragment sample can be obtained. Various electrophoresis techniques are available, such as gel electrophoresis and capillary electrophoresis. In electrophoresis, amplified DNA fragments are migrated in a charged electrophoretic path, and thereby DNA fragments with different lengths can be separated. Electrophoresis is a sample isolation technique utilizing the phenomenon in which DNA fragments with different lengths migrate in an electrophoretic path at different migration speed (the longer the DNA fragment, the lower the migration speed).

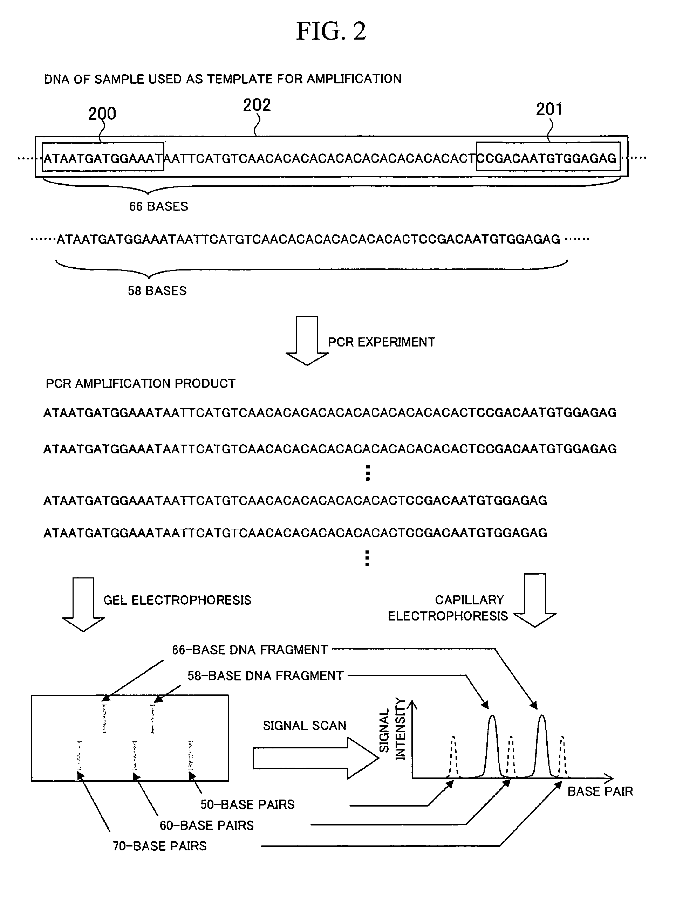

Fig. 2 is a schematic view of an experimental procedure for extracting and amplifying a DNA fragment containing a microsatellite by PCR technique and gel electrophoresis. Firstly, a pair of primer sequences 200 and 201, which sandwich a target microsatellite, is designated, and a genome region 202 containing the microsatellite and primer sequences is amplified in a PCR experiment. The example shown in Fig. 2 is a heterozygote in which microsatellites on two homologous chromosomes have different numbers of microsatellite repetitions. Since the lengths of these two microsatellites vary, 2 types of PCR amplification products of different lengths, DNA fragments of 66 and 58 bases, are obtained. Having been applied on a plate gel and caused to migrate for a certain period of time, these 2 types of PCR amplification products can be separated on the basis of the length of DNA fragment. Each of the DNA fragments is marked with a fluorescent dye. Intensity and location of a fluorescent signal of each of the DNA fragments are detected after electrophoresis, and a graph can be plotted, as shown Fig. 2, with length of DNA fragment (migration distance) on the x-axis and fluorescent signal intensity (amount of DNA fragment) on the y-axis.

In the electrophoresis, DNA fragments with known lengths (called size markers) can also be applied on a gel together with PCR amplification products, and then the fluorescent signals from these size markers are detected. Accordingly, it is possible to estimate the length of each PCR amplification product by referring to the locations of the size markers detected on the gel.

Although the example above employs an experimental technique using gel electrophoresis, capillary electrophoresis may also be employed for the same purpose. In the capillary electrophoresis, a sample is caused to migrate through a fine tube filled with gel. The period of time taken for each sample to migrate for a certain distance (normally to the end of the capillary) is measured, and thereby, the length of a DNA fragment in the sample can be obtained. In the capillary electrophoresis, sample detection is usually performed by a fluorescent signal detector provided at the end of a capillary, not by scanning the fluorescent signals of the sample in a gel.

### Noises generated during PCR and electrophoresis experiments

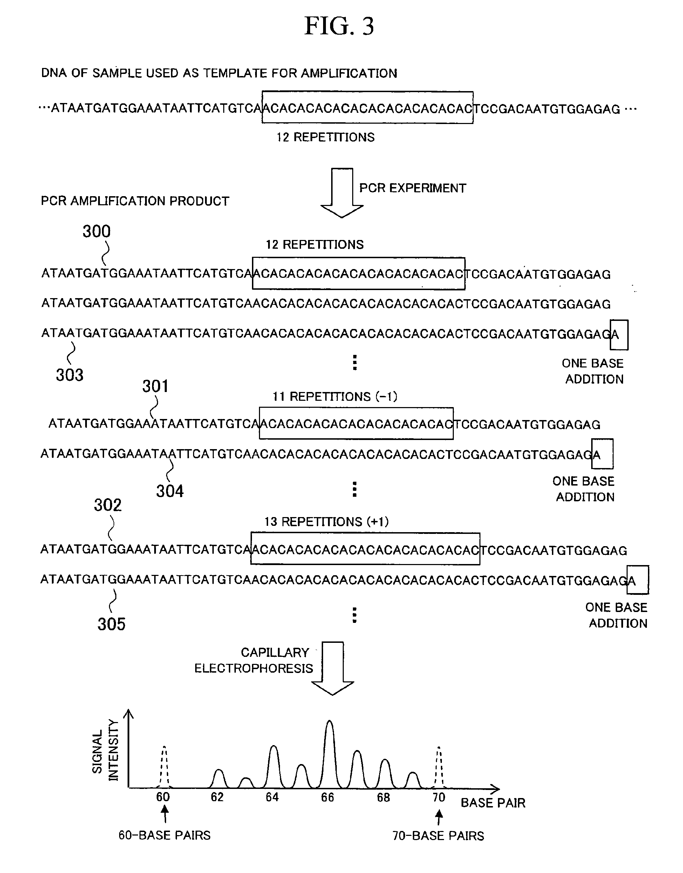

The experimental result shown in Fig. 2 above can be obtained when PCR technique and electrophoresis experiments are conducted under ideal conditions. In actual experiments, various noises may arise. A stutter peak and +A peak, which are typical noises generated during PCR and electrophoresis experiments, will be explained in this s section with referring to Fig. 3.

In order to simplify explanations, Fig. 3 shows only a 66-base DNA fragment (including a microsatellite in which "AC" is repeated 12 times) shown in Fig. 2.

A stutter peak is a noise caused by a phenomenon in which the number of microsatellite repetitions in a target DNA fragment is either increased or decreased due to a slipped-strand mispairing during PCR. In a fluorescence analysis, such a DNA fragment including either more or less microsatellite repetitions is observed as a noise peak.

As shown in Fig. 3, in addition to a DNA fragment containing a normal microsatellite with 12 repetitions of "AC," DNA fragment 301 and 302 containing an abnormal microsatellite with 11 and 13 repetitions of "AC," respectively, may be produced. Such abnormal fragments are observed as stutter peaks in a fluorescence analysis in many cases. A greater increase or decrease in the number of repetitions may also occur. Hence, it is possible that a DNA fragment which is shorter or longer than a normal DNA fragment by an integral multiple of a unit length, is produced in addition to a DNA fragment of the same length as the template DNA (66 bases).

A +A peak is a noise caused by a phenomenon in which an extra base (usually A) is added to a replicated DNA fragment during PCR. In fluorescence analysis, such a DNA fragment with an additional base is observed as a noise peak. As shown in Fig. 3, in addition to a DNA fragment 303 which includes a normally replicated DNA fragment 300 having an extra base added, abnormal DNA fragments 304 and 305 which each include the abnormally replicated DNA fragments 301 and 302 with a smaller or larger numbers of microsatellite repetitions, due to slipped-strand mispairing, and further with having an extra base added, respectively may be produced. These DNA fragments 303, 304, and 305 with an extra base are each observed as different +A peaks in a fluorescence analysis.

In the graph showing a result of the fluorescence analysis in Fig. 3, the peak of the DNA fragment of 66 bases, whose length is equal to that of the template DNA fragment, is the one which is supposed to be observed (hereafter referred to as "a true peak"), and other peaks are all noise peaks. It is observed that stutter peaks appear at intervals of the unit length of the microsatellite (at 62, 64, and 68 base sites) from the true peak. It is also observed that +A peaks appear at more distant sites (at 63, 65, 67, and 69 base sites) than each of the true peak and the stutter peaks by one base pair. To be more precise, the +A peaks observed at 63, 65, 67, and 69 base sites correspond to DNA fragments of 62, 64, 66, and 68 bases each having an extra 1 base added, respectively.

In the following section, a true peak or stutter peak derived from a DNA fragment to which an extra base is added to produce a +A peak is called "an original peak."

Microsatellites on a pair of genomes are either homozygous or heterozygous. The waveform graph of a fluorescent signal of an extracted DNA fragment will be very different depending on whether the DNA fragment is homozygous or heterozygous. While only one true peak is to be observed for a homozygote, two true peaks are to be observed for a heterozygote. However, as clearly shown in the fluorescence analysis result in Fig. 3, many peaks may be observed even for a homozygote. Hence, a problem arises in determining whether an extracted DNA fragment is homozygous or heterozygous according to a waveform graph of the fluorescent signal and the number of peaks.

In PCR and electrophoresis experiments, it is extremely important to discriminate a true peak from other peaks among multiple peaks observed in fluorescence analysis. Among noise peaks described above, a stutter peak can be precisely discriminated according to some principles thereof, such as "a stutter peak is lower than a true peak," by the methods disclosed in U.S. Pat. Nos. 5,541,067; 5,580,728; 5,876,933; 6,054,268; and 6,274,317, and Perlin, M. W. et al., "Toward Fully Automated Genotyping: Allele Assignment, Pedigree Construction, Phase Determination, and Recombination Detection in Duchenne Muscular Dystrophy," Am. J. Hum. Genet. 55, 1994, p777-787; Perlin, M. W. et al., "Toward Fully Automated Genotyping: Genotyping Microsatellite Markers by Deconvolution," Am. J. Hum. Genet. 57; 1995, p1199-1210; Palsson, B., et al., "Using Quality Measures to Facilitate Allele Calling in High-Throughput Genotyping," Genome Research 9, 1999, p1002-1012; and Stoughton, R., et al., "Data-adaptive algorithm for calling alleles in repeat polymorphisms," Electrophoresis 18, 1997, p1-5. Softwares, such as "TrueAllele" by Cybergenetics, Co., "SAGA" by LI-COR Biosciences, and "GenoTyper" and "GeneMapper" by Applied Biosystems (all are trade names), are known for performing processing of discriminating and eliminating stutter peaks. Among the noise peaks, as for a +A peak, it is possible to estimate the tendency of occurrence for each marker, for example, by using a method disclosed in Matsumoto, T., et al., "Novel algorithm for automated genotyping of microsatellites," Nucleic Acids Research Vol.32, No.20, 2004, p6069-6077. The method utilizes the fact that occurrence patterns of +A peaks observed in experiments conducted simultaneously under the same condition, using the same marker are in the same range; in other words, the height ratios between an original peak and its corresponding +A peak are in the same range.

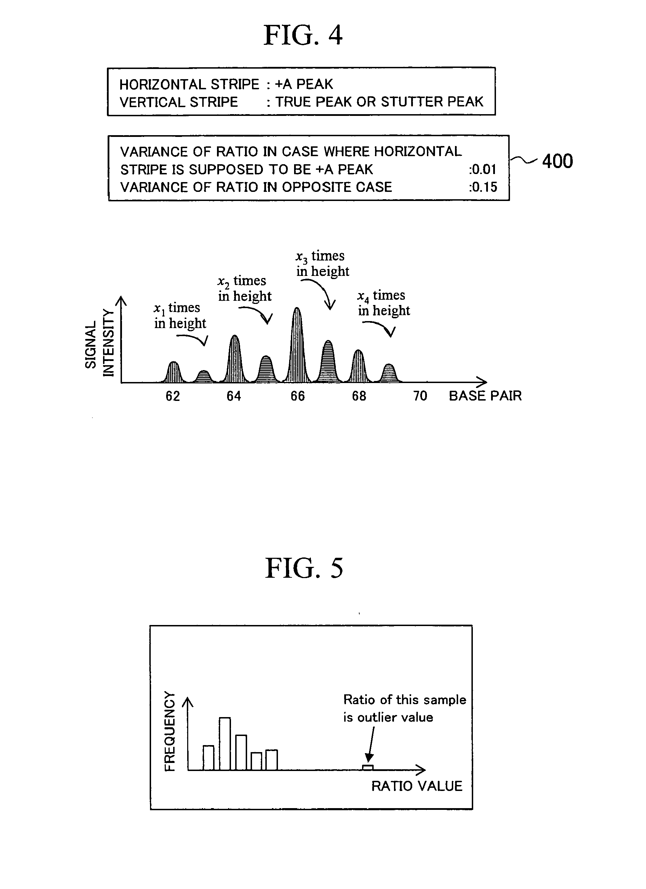

As shown by reference numeral 400 in Fig. 4, a peak interpretation is sought such that the ratio between each of the original peaks, such as a true peak and stutter peaks, and each of the corresponding +A peaks stays approximately constant in each sample. At the same time, as shown in Fig. 5, it is examined whether or not the ratio varies largely among multiple samples including the same marker, and any sample with an outlier ratio value will be eliminated. The ratio value obtained in this process is used as an estimating result of a +A peak occurrence pattern for the marker.

### SUMMARY OF THE INVENTION

The above-described conventional methods for discriminating noise peaks work well under some experimental conditions; however, in the following cases, they have a problem of being unable to estimate +A peaks properly.

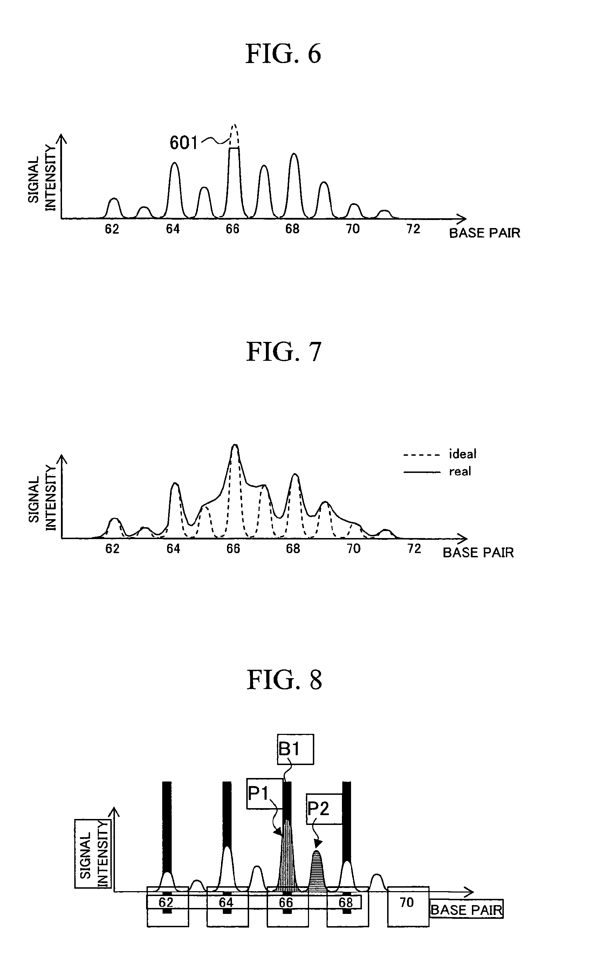

Fig. 6 shows a first case in which intensity of a fluorescent signal indicated by reference numeral 601 is so strong that detection limit for signal intensity is exceeded. A dashed line 601 in Fig. 6 shows an estimated waveform on the premise that the fluorescent signal is properly measured. Fig. 6, in which the vertical axis indicates fluorescent signal intensity observed by a detector, shows an example in which detection limit of a detector is exceeded due to an exceedingly intense fluorescent signal. The fluorescent signal became exceedingly intense because an amount of 66-base fragments contained in a sample was larger than expected. Under such a condition, the top part of the peak in the waveform data may be missing, and the peak height thereof may be measured shorter than that actually is. In such a case, even if the ratio between an original peak, such as a true peak and a stutter peak, and the +A peak thereof actually stays approximately constant, a large variety of ratios may be observed.

Fig. 7 shows a second case in which acquired observational data include peaks that are unable to be completely separated from each other. A dashed line in Fig. 7 shows an estimated waveform on the premise that the peaks have been able to be completely separated. Since a migration speed in electrophoresis varies among fragments with different lengths, it is supposed to be able to separate DNA fragments by length. However, if separation is not successful, the waveform as shown by the solid line may be observed. In such a case, if a high peak and a low peak are located next to each other with respect to the direction of base pair in the graph, the low peak may be hidden under the skirt of the high peak, and therefore may not be accurately observed (In this particular case, the 65-base peak cannot be observed due to the 66-base peak). Hence, the ratio of heights between an original peak (64-base peak) and the +A peak thereof (65-base peak) may be calculated to be zero, which is far off from the real value that should be calculated to be. For this reason, as described in the first case, even if the ratio between an original peak, such as a true peak and a stutter peak, and the +A peak thereof in nature stays approximately constant, a large variety of ratios may be observed.

In addition, regarding the phenomenon in which peak observation is disturbed due to poor separation, the lower peak between neighboring right and left peaks is likely to be more difficult to be observed. In the example shown in Fig. 7, between neighboring peaks on the right and left of the 66-base peak, the 65-base peak is lower and almost completely unable to be observed, while the 67-base peak can be detected as a peak with a small portion of the peak top shown in the graph.

A third case involves problems of being unable to perform experiments simultaneously due to operation schedules of experimental equipment and other similar factors. The +A peak occurrence pattern is susceptible to temperature in a laboratory and any slight time lag between PCR and electrophoresis. With the presence of any of these factors affecting the +A peak occurrence pattern, the +A peak occurrence pattern may vary even if the same marker is used. For example, suppose that an original peak is higher than the +A peak thereof in the first experiment, but is lower in the second experiment. According to the conventional method for estimating +A peak, it will be concluded that the ratio varies largely among samples using the same marker; thus, it will be determined that the sample obtained in the second experiment has an outlier value and is not suitable for estimating a +A peak occurrence pattern. As a result, a wrong estimation, "an original peak is higher than the +A peak thereof in all samples," would be made. The use of such a wrong estimation leads to a problem that the +A peak derived from the sample in the second experiment is determined to be a true peak.

In the above-described cases, if conventional methods are employed, it is impossible to properly determine whether or not a peak is a noise peak; thus, time has to be spent to visually check a determination result and to manually perform necessary corrections. This becomes a bottleneck in analysis processing.

The present invention aims at improving user convenience in an experiment using a microsatellite marker.

As more experiments have been conducted utilizing microsatellite markers, findings for individual markers are being accumulated, and therefore, the number of markers with known alleles is increasing. Although such findings involve two difficulties which will be described in the following section, they can be utilized as reference information for automatic genotype determination.
1) The first difficulty is that the finding for each of the markers is a list of alleles which can appear, and therefore, the finding does not necessarily directly indicate genotype of the sample which an experimenter is analyzing.
2) The second difficulty is that findings of a marker which has a true peak lower than the +A peak thereof may not be a list of alleles (the fragment length of a true peak) which can appear, but a list of the alleles above having one extra base added (the fragment length of the +A peak of the true peak).

Hereafter, a peak of any allele which can appear (a peak being still unknown whether the peak is a true peak or the +A peak thereof) is referred to as a "reported peak."

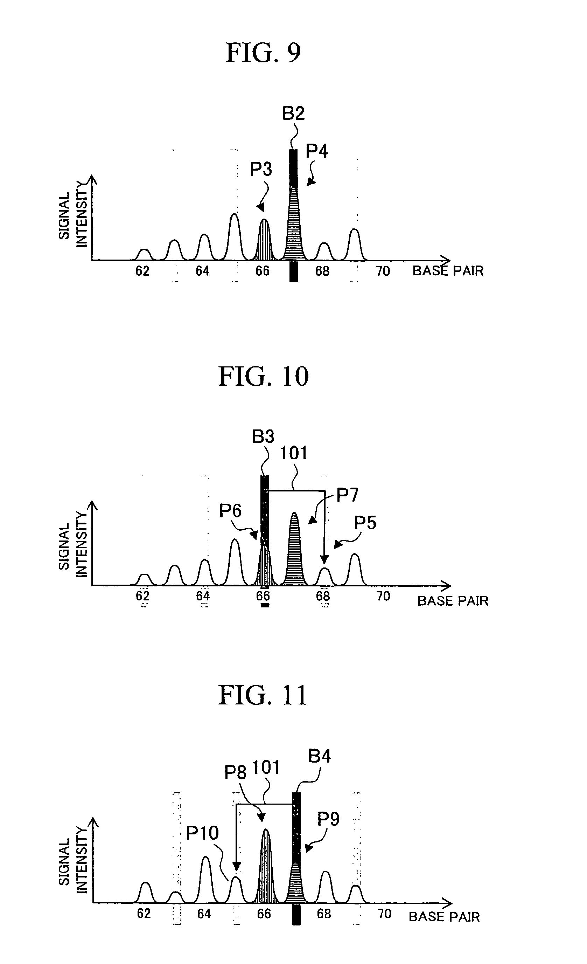

The present invention is characterized by performing accurate estimation of a +A peak occurrence pattern by use of the following functions and the above-described findings, in order to solve the above-described problems.
(1) Function 1: To determine whether or not each of the samples is suitable to be used for examination of a +A peak occurrence pattern.
   Function 1-1: In Function 1, a positional relationship of the highest peak with each of the neighboring reported peaks thereof is investigated, and then any sample which has been determined to be unsuitable to be used for examination of a +A peak occurrence pattern is eliminated. If no reported peak is located within the range of one base pair from the highest peak, it is highly possible that the highest peak is neither a true peak nor the + A peak thereof, but is a noise peak which appears incidentally. Hence, this sample is determined to be unsuitable to be used for examination of a +A peak occurrence pattern. If a reported peak is located in the vicinity of the highest peak, with a distance not equivalent to the unit length of a sample from the highest peak, the sample is determined to be unsuitable to be used for examination of a +A peak occurrence pattern. The reason for this decision is that a heterozygous sample having two true peaks distant from each other with a distance not equivalent to the unit length may have both a +A peak derived from each of the alleles and the original peak overlapping with each other, and therefore, it is impossible to calculate the ratio accurately.
(2) Function 2: To determine which reported peak should be determined to be derived from each of the samples which have been determined to be suitable to be used for examination of a +A peak occurrence pattern in Function 1, by investigating a positional relationship of the highest peak with the neighboring reported peaks thereof. In Figs. 8 to 11, vertically-striped peaks P1, P3, P6, and P8 are true peaks, and horizontally-striped peaks P2, P4, P7, and P9 are +A peaks of the true peaks. Gray lines indicate reported alleles, and dark gray lines B1 to B4 indicate peaks which have been determined to be the reported peaks which the individual samples have. As shown in Figs. 8 and 9, when the highest peaks P1 and P4 correspond with reported peaks Bland B2, respectively, it is possible to determine that the highest peaks P and P4 are the reported peaks of the respective samples. The reason for this determination is that, since "a stutter peak is lower than a true peak," and "the ratio of heights between an original peak and the +A peak thereof is in the same range," as described in the Description of the Related Art section above, the highest peak in a waveform is either a true peak or its +A peak thereof.
   In the case, as shown in Fig. 10, where the peak P6 one base pair distant from the highest peak P7 on the left side thereof corresponds with the reported peak B3, and the peak P5 one base pair distant from the highest peak P7 on the right side thereof either does not correspond with a reported peak or is lower than the peak P6 one base pair distant from the highest peak P7 on the left side thereof, it can be determined that the peak P6 one base pair distant from the highest peak P7 on the left side thereof is the reported peak of this sample. The reason for this determination is that, if it is taken that, in a sample having the unit length of 2 bases, the peaks one base pair distant from the highest peak on the left and right sides thereof are a true peak and a stutter peak which is one unit length longer than the true peak, respectively, the above-described case corresponds with the principle "a stutter peak is lower than a true peak." In the case where the unit length is larger than or equal to 3 bases, since reported peaks are located at 3 base pair intervals, it never happens that both peaks on the right and left sides of the highest peak correspond with reported peaks. Hence, in such a case, a peak which corresponds with a reported peak on the left side should be determined to be the reported peak of the sample. In the case, described in the Description of the Related Art section above, where peaks cannot be observed due to poor separation, observation of the peak P5 at the 68 base site becomes impossible before that of the peak P6 at the 66 base site in Fig. 10. If the peak P5 at the 68 base site is no longer observed and the peak P5 at the 66 base is observed, it is possible to precisely determine a relationship between the peaks at the 66 base and 68 base sites in terms of peak height. Hence, this determination technique has been proven to be suitable for solving the problem in which peaks cannot be observed due to poor separation.
   In the case, as shown in Fig. 11, where the peak P9 one base pair distant from the highest peak P8 on the right side thereof corresponds with a reported peak, and the peak P10 one base pair distant from the highest peak P8 on the left side thereof either does not correspond with a reported peak or is lower than the peak P9 one base pair distant from the highest peak P8 on the right side thereof, it can be determined that the peak P9 one base pair distant from the highest peak P8 on the right side thereof is the reported peak of this sample. The reason for this determination is that, as described for the case shown in Fig. 10, if it is taken that, in a sample having the unit length of 2 bases, the peak P9 on the right side of the highest peak P8 is a +A peak of a true peak and the peak P10 on the left side is the +A peak of a stutter peak that is one unit length shorter than the true peak, the above-described case corresponds with the principles "a stutter peak is lower than a true peak" and "the ratio of heights between an original peak and the +A peak thereof is in the same range." As described above by referring to the case shown in Fig. 10, this determination technique has also been proven to be suitable for solving the problem in which peaks cannot be observed due to poor separation.
   By using this technique, it is possible to correctly determine which is the reported peak of a sample in cases where the true peak is higher than its +A peak thereof (in the cases of Figs. 8 and 11), where the true peak is lower than its +A peak thereof (in the cases of Figs. 9 and 10), where the reported peak is the true peak (in the cases of Figs. 8 and 10), and where the reported peak is the +A peak (in the cases of Figs. 9 and 11).
(3) Function 3: To determine whether the reported peak is a true peak or the +A peak thereof.
   (3-1) Function 3-1: In Function 3, it has been determined, for individual sample, whether the reported peak is the true peak or its +A peak thereof. Then, determination is made for the whole data by majority vote among samples. By adopting this procedure, it is possible to reduce the probability of making an erroneous determination for the whole data even if some samples are mistakenly determined.
   (3-2) Function 3-2: In Function 3-1, for a heterozygote, it has been determined whether a reported peak of each of the two alleles is a true peak or its +A peak thereof. Then, if determinations for both alleles agree with each other, the determination is adopted. If not, a determination is withheld.
      By adopting this procedure, even if an erroneous decision is made for one allele, the probability of an erroneous decision for the sample can be reduced. Regarding whether or not a sample is a heterozygote, a sample is determined to be a heterozygote in cases where two peak clusters with a single peak are observed, and where one bimodal cluster of peaks is observed, as described in Matsumoto, T., et al.
   (3-3) Function 3-3: In the above-described Function 3-2, it has been determined whether the reported peak is a true peak or its +A peak thereof by investigating a positional relationship of the highest peak with the neighboring reported peaks. As the cases shown in Figs. 8 and 9 described in Function 2, in the case where the highest peak corresponds with a reported peak, determination is performed according to a method to be described in Function 3-4. As in Fig. 10, in the case where the peak one base pair distant from the highest peak on the left side thereof corresponds with a reported peak, and the peak one base pair distant from the highest peak on the right side thereof either does not correspond with a reported peak or is lower than the peak one base pair distant from the highest peak on the left side thereof, it can be determined that the reported peak is a true peak. As shown in Fig. 11, in the case where a peak one base pair distant from the highest peak on the right side thereof corresponds with a reported peak, and the peak one base pair distant from the highest peak on the left side thereof either does not correspond with a reported peak or is lower than the peak one base pair distant from the highest peak on the right side thereof, the reported peak is determined to be a +A peak.
   (3-4) Function 3-4: In Function 3-3, as shown in Figs. 8 and 9, in the case where the highest peak corresponds with a reported peak, it is determined whether the reported peak is a true peak or its +A peak thereof by comparing heights of the peaks one base pair distant from the highest peak on the right and left sides thereof.

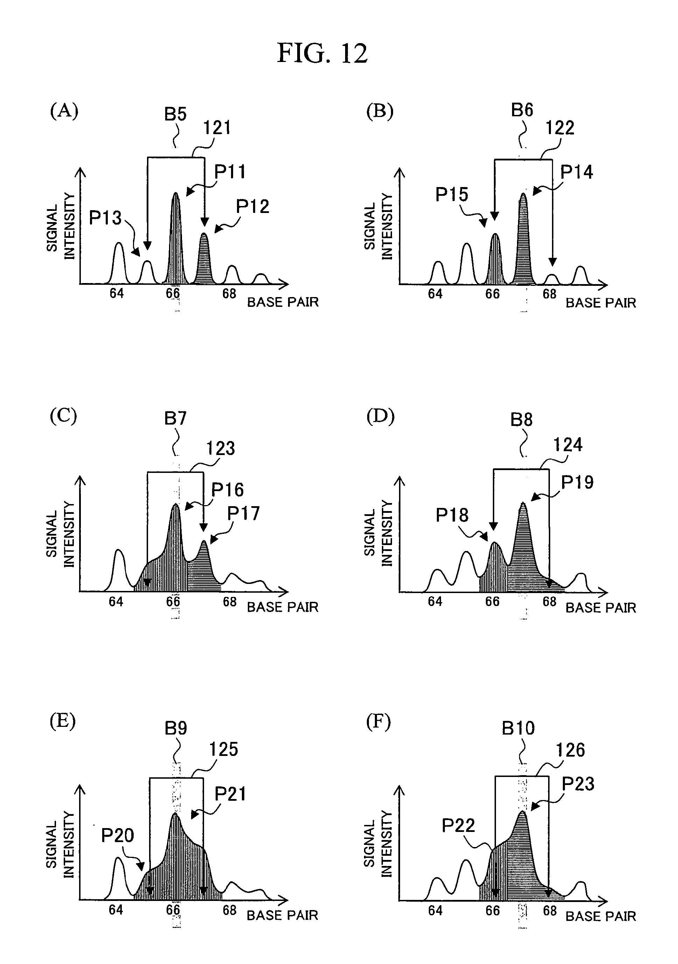

      In Fig. 12, gray lines B5 to B10 indicate reported peaks, vertically-striped peaks P11, P 15, P16, P 18, P20, and P22 are true peaks, horizontally-striped peaks P12, P 14, P 17, P 19, and P23 are +A peaks of the corresponding true peaks. In three graphs Figs. 12 (A), (C), and (E) on the left side, the reported peak corresponds with the true peak (the true peak is higher than the +A peak thereof). In three graphs Figs. 12 (B), (D), and (F) on the right side, a reported peak corresponds with the +A peak of the true peak (the true peak is lower than the +A peak thereof).
      Figs. 12 (A) and (B) on the top row show good separation among all peaks, and Figs. 12 (C) and (D) in the middle row show slightly poor separation between the highest peak and the lower one of the peaks on the right and left sides thereof (they are merged together). Figs. 12 (E) and (F) show poor separation between the highest peak and both of the peaks on the right and left sides (they are merged together). As shown in such cases, an extremely large number of types of waveform can be observed in terms of peak separation. However, it is basically possible to discriminate between waveforms on the right and left by comparing heights of the peaks one base pair distant from the highest peak on the right and left sides thereof.
      To be more precise, the peak one base pair distant from the highest peak on the right side thereof is higher than the peak one base pair distant from the highest peak on the left side thereof in two waveforms shown in Figs. 12 (A) and (C), and, conversely, lower in two waveforms shown in Figs. 12 (B) and (D). In the waveforms shown in Figs. 12 (E) and (F), neither of the peaks one base pair distant from the highest peak on the right and left sides is detected, and therefore the height is determined to be zero. In such cases, determination whether a reported peak is a true peak or the +A peak thereof is withheld. This is because, even if a determination is withheld in some samples, it is still possible to make a determination for the whole, as described in Function 3-1, on the basis of determinations made for other samples.
      By utilizing Functions 3-3 and 3-4, it is possible to correctly determine whether a reported peak is a true peak or the +A peak thereof in cases where the true peak is higher than the +A peak thereof (in the cases of Figs. 8 and 11), where the true peak is lower than the +A peak thereof (in the cases of Figs. 9 and 10), where the reported peak is the true peak (in the cases of Figs. 8 and 10), and where the reported peak is the +A peak thereof (in the cases of Figs. 9 and 11).
   (3-5) Function 3-5: In addition to the methods described in Functions 3-3 and 3-4, in the case where a unimodal cluster of peaks is observed, it is determined whether a reported peak is a true peak or its +A peak thereof by investigating increase or decrease relationship in height between peaks being located at intervals of the unit length as described in the following section.

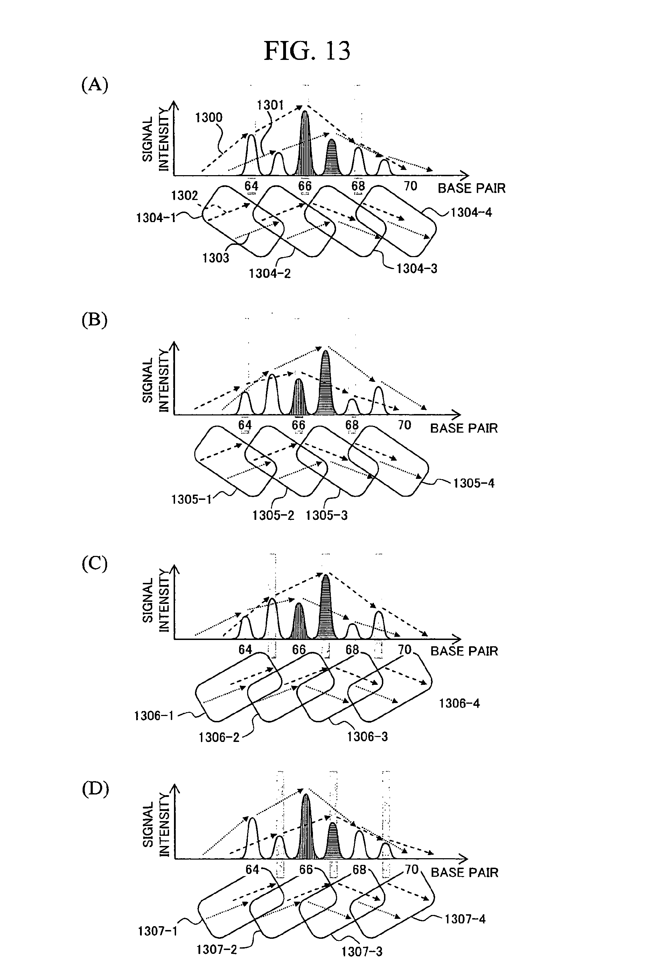

As shown in Fig. 13, vertical gray lines indicate sites the unit length distant from a reported peak on the right and left sides thereof, a vertically-striped peak at the 66 base site is a true peak, and a horizontally-striped peak on the right side of the true peak is the +A peak of the true peak. Dashed arrows, such as the one indicated by reference numeral 1300, show increase or decrease relationships in height between peaks being located at intervals of the unit length from the reported peak, and dotted arrows, such as the one indicated by reference numeral 1301, show increase or decrease relationships in height between peaks being located at intervals of the unit length from the site one base pair distant from the reported peak on the right side thereof.

Dashed arrows, such as the one indicated by reference numeral 1302, and dotted arrows, such as the one indicated by reference numeral 1303, being located below the waveforms each show increase or decrease relationships alone on the arrows indicated by reference numeral 1300 and 1301 and the like, without their actual increased or decreased quantities. Gray oval figures, such as the ones indicated by reference numerals 1304-1 to 1304-4, have been drawn to surround adjacent arrows so that they have similar increase or decrease relationships within each oval figure.

For example, in the waveform shown in Fig. 13 (A), an arrow indicated by reference numeral 1300 shows increase or decrease relationship in height between the peaks at the 62 and 64 base sites. As this arrow goes diagonally right up, the peak at the 64 base site is higher (in this case, no peak at the 62 base site is observed; thus, the height at the position is considered to be zero.). Similarly, other dashed arrows on peaks indicate increase or decrease relationships in height between the peaks at the 64 and 66 base sites, at the 66 and 68 base sites, and at the 68 and 70 base sites. The arrow indicated by reference numeral 1302 which corresponds to that by reference numeral 1300 and is substantially the same, is shown to clearly indicate that Function 3-5 is focusing only on increase or decrease relationship in height between the peaks, not peak heights thereof. An oval figure indicated by reference numeral 1304-1 surrounds both an dashed arrow indicated by reference numeral 1302 and a dotted arrow indicated by reference numeral 1303, and shows that increase or decrease relationships in height, indicated by these vertically adjacent arrows correspond with each other (both arrows go diagonally right up).

When only original peaks (a group of peaks being located at intervals of a unit length from a true peak on the right and left sides thereof) and only +A peaks (a group of peaks being located at intervals of a unit length from a +A peak of a true peak, on the right and left sides of the +A peak) are each taken out from a peak cluster for examination, increase or decrease relationship in height between peaks are supposed to be similar according to the principle "the ratio of heights between an original peak and the +A peak thereof is in the same range." In addition, since each of the +A peaks is located at the site one base longer than the corresponding original peaks, the +A peaks and the original peaks are supposed to be overlapping each other off by length of a single base. By drawing substantially oval figures, such as the one indicated by reference numeral 1304-1, so as to surround both a dashed arrow, such as the one indicated by reference numeral 1302, and a dotted arrow, such as the one indicated by reference numeral 1303, sequentially in the direction of increasing base pair, it can be exhibited that two peak clusters with similar increase or decrease relationships in height are overlapping each other off by length of a single base.

Waveforms in Figs. 13 (A) and (B) show the case where a reported peak is a true peak, while waveforms shown in Figs. 13 (C) and (D) show the case where a reported peak is a +A peak of a true peak. By simply acquiring increase or decrease relationships on the resultant dashed and dotted arrows, drawing an oval figure to surround these arrows according to the increase or decrease relationships, and visually observing the direction of the oval figure, it is possible to determine whether a reported peak is a true peak or the +A peak thereof irrespectively of which one of a true peak and the +A peak thereof is higher.

In other words, as the waveforms shown in Figs. 13 (A) and (B), when the direction of an oval figure is diagonally left up (in the case where increase or decrease relationships correspond with each other when a pair is made with a dashed arrow on the left and a dotted arrow on the right), peaks which have been investigated about increase or decrease relationships in height indicated by the dashed arrows are original peaks, and peaks which have been investigated about increase or decrease relationships in height indicated by the dotted arrows are the +A peaks. Conversely, as the waveforms shown in Figs. 13 (C) and (D), when the direction of an oval figure is diagonally right up (in the case where increase or decrease relationships correspond with each other when a pair is made with a dotted arrow on the left and a dashed arrow on the right), peaks which have been investigated about increase or decrease relationships in height indicated by the dotted arrows are original peaks, and peaks which have been investigated about increase or decrease relationships in height indicated by the dashed arrows are the +A peaks.

If determinations in Functions 3-3, 3-4, and 3-5 do not correspond with each other, it is suggested that an erroneous determination has been made in any of these functions; thus, a determination is withheld. The following three cases are expected to be as a unimodal cluster of peaks.

A first case is a waveform of a homozygote. A second case is a waveform of a heterozygote including 2 alleles being located sufficiently distant from each other, and 2 unimodal clusters of peaks are observed in this case. A third case is a waveform of a heterozygote including 2 alleles being located extremely close to each other (e.g. only 1 unit length apart). In the first and second cases, since only a single reported peak is included in a peak cluster, it is possible to calculate the ratio properly. In the third case, since the possibility of a sample being a heterozygote having two true peaks being located with a distance that is not equivalent to a multiple of the unit length is eliminated by using Function 1-1, it is also possible to calculate the ratio properly.

Hence, as shown in Fig. 4, since the ratio between an original peak and its +A peak thereof is approximately constant within a single sample, it is possible to adopt the ratio of heights between a peak formed by overlapping of both a stutter peak derived from a first true peak and a second true peak and a peak formed by overlapping of the +A peaks of both the stutter peak and the second true peak.

According to the present invention, it is possible to estimate a +A peak occurrence pattern with a supplementary input of reported peak data, and to obtain and display sample data which have been used as grounds for estimating a +A peak occurrence pattern.

### BRIEF DESCRIPTION OF THE DRAWINGS

Fig. 1 is a view explaining microsatellites observed in a genome.
Fig. 2 is a schematic view showing an experimental procedure for extracting and amplifying DNA fragments containing microsatellites by PCR technique and electrophoresis.
Fig. 3 is a view explaining a stutter peak and a + A peak, which are typical noises, appearing in an experimental procedure using PCR technique and electrophoresis.
Fig. 4 is a view explaining a method for determining which peaks are an original peak and the +A peak thereof on the basis of the ratios between original peaks and the +A peaks thereof.
Fig. 5 is a chart showing an example in which there is an outlier among multiple samples with the same marker in terms of height ratios between original peaks and the +A peaks thereof.
Fig. 6 shows a waveform which appears in a waveform data of a fluorescence analysis result in the case where a fluorescent signal is too intense to stay within measurement limit.
Fig. 7 is a view showing a waveform which appears in a waveform data of a fluorescence analysis result in the case where peaks were not completely separated.
Fig. 8 is a view showing a positional relationship among a true peak, the +A peak thereof, and reported peaks.
Fig. 9 is a graph showing a positional relationship among a true peak, the +A peak thereof, and reported peaks.
Fig. 10 is a view showing a positional relationship among a true peak, the +A peak thereof, and reported peaks, and a relationship between heights of the right and left peaks.
Fig. 11 is a view showing a positional relationship among a true peak, the +A peak thereof, and reported peaks, and a relationship between heights of the right and left peaks.
Fig. 12 is views showing a positional relationship among a true peak, the +A peak thereof, and a reported peak, and a relationship between heights of the right and left peaks in each of the cases where peaks have been separated and where peaks have not been completely separated.
Fig. 13 is a view showing increase or decrease relationships in height between peaks in the vicinity of reported peaks.
Fig. 14 is a view showing a screen displaying a result of a determination whether or not a sample is suitable to be used for examining a +A peak occurrence pattern.
Fig. 15 is a view showing a screen displaying a result of a determination which reported peak is derived from a sample.
Fig. 16 is a view showing a result of a determination whether or not a reported peak is a true peak or the +A peak thereof for the whole data.
Fig. 17 is a view showing a result of a determination whether or not a reported peak is a true peak or the +A peak thereof based on a single sample.
Fig. 18 is a view showing a result of a determination whether a reported peak is a true peak or the +A peak thereof.
Fig. 19 is a view showing a result of a determination whether a reported peak is a true peak or the +A peak thereof.
Fig. 20 is a view showing a result of a determination whether a reported peak is a true peak or the +A peak thereof.
Fig. 21 is a functional block diagram showing an outline of an internal configuration of a sample genotype estimation apparatus which is established as an embodiment of the present invention.
Fig. 22 is a view showing a data structure of experimental data 2115 included in data memory 2106 in the sample genotype estimation apparatus described in Fig. 21.
Fig. 23 is a flowchart showing a flow of processing in the present invention
Fig. 24 is a flowchart showing detailed processing for examining a +A peak occurrence pattern based on reported peak data.
Fig. 25 is a flowchart showing detailed processing for investigating which reported peak is derived from a sample.
Fig. 26 is a flowchart showing detailed processing for investigating whether a reported peak is a true peak or its +A peak thereof by examining reported peaks in the vicinity of the highest peak.
Fig. 27 is a flowchart showing detailed processing for determining a reported peak is a true peak or the +A peak thereof by examining peaks one base pair distant from the highest peak on the right and left sides thereof.
Fig. 28 is a flowchart showing detailed processing for investigating whether a reported peak is a true peak or the +A peak thereof by examining increase or decrease relationships in height between peaks in the vicinity of the reported peak.

### DESCRIPTION OF THE PREFERRED EMBODIMENT

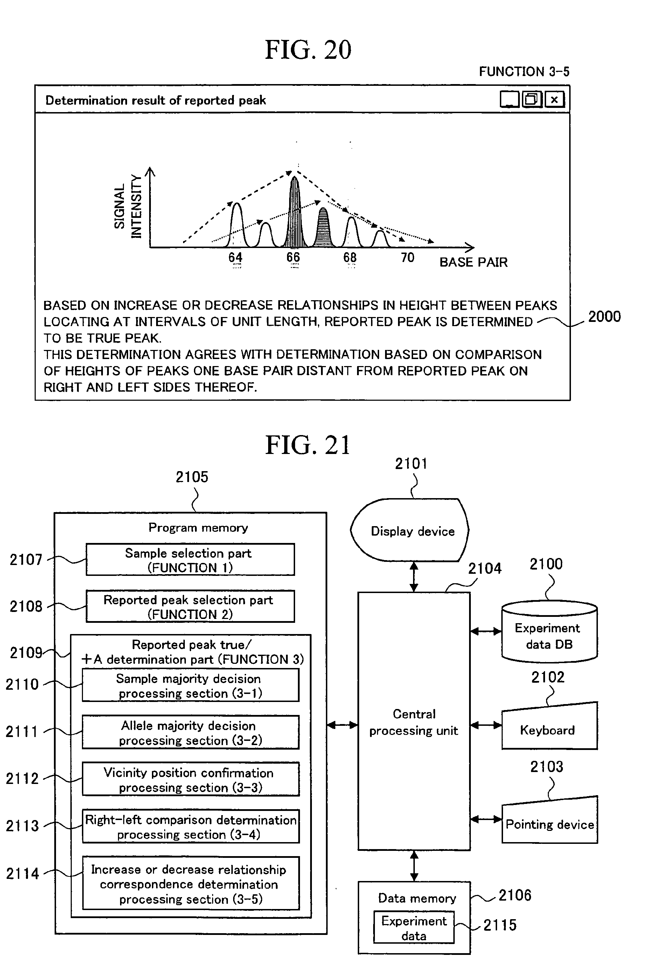

In the following section, a gene information processing technique according to an embodiment of the present invention will be explained in detail by referring to the attached drawings. Figs. 21 to 28 are drawings showing a configuration example of a gene information processing apparatus according to the embodiment of the present invention. In these drawings, any parts sharing the same reference numeral indicate the same component, and the basic configuration and operation are in the same range.

Fig. 21 is a functional block diagram showing an outline of an internal configuration example in the gene information processing apparatus according to the embodiment of the present invention. The gene information processing apparatus includes: a database 2100 storing experimental data 2115 containing data obtained from experiments; a display device 2101 for displaying data; a keyboard 2102 and a pointing device (operating portion) 2103 such as a mouse, for performing operations on displayed data, such as selecting a menu; a central processing unit (CPU) 2104 for performing required arithmetic processing, control processing and the like; a program memory 2105 storing programs required for processing of the CPU 2104; and a data memory 2106 for storing data required for processing in the CPU 2104. An output to or in place of the display device 2101, a printer output, an audio output or the like may be performed.

The program memory 2105 includes a sample selection part 2107 which performs above-described Function 1, a reported peak selection part 2108 which performs above-described Function 2, and a reported peak true/+A determination part 2109 which performs above-described Function 3. The reported peak true/+A determination part 2109 includes a sample majority decision processing section 2110 which performs above-described Function 3-1, an allele majority decision processing section 2111 which performs above-described Function 3-2, a vicinity position confirmation processing section 2112 which performs above-described Function 3-3, a right-left comparison determination processing section 2113 which performs above-described Function 3-4, and a increase or decrease relationship correspondence determination processing section 2114 which performs above-described Function 3-5. The data memory 2106 includes the data 2115 which have been obtained from experiments. These can be operated in a general computer system.

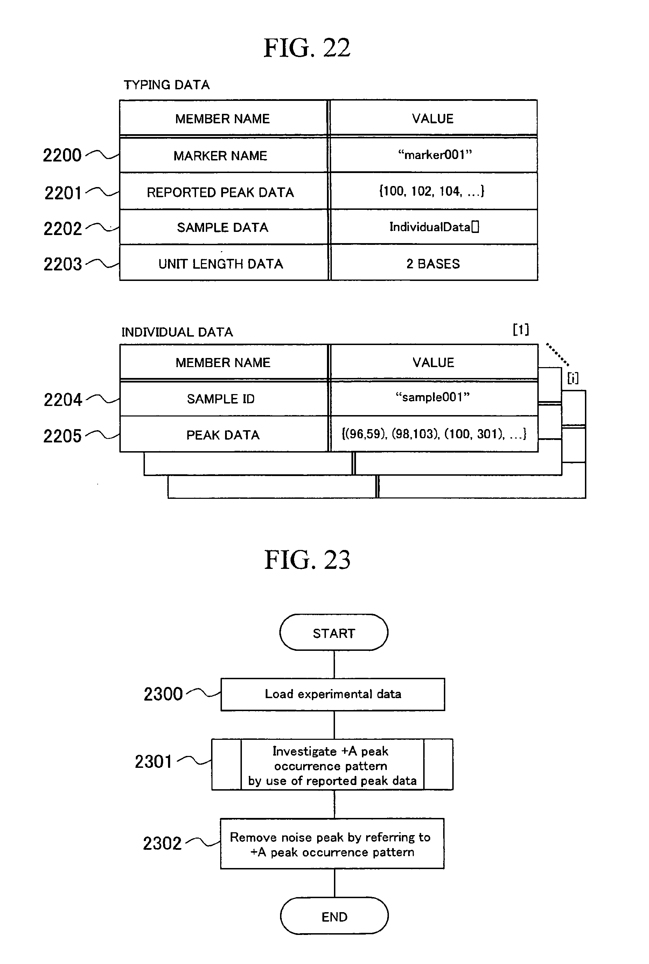

Fig. 22 shows a data structure example of the experimental data 2115 included in the data memory 2106. The data structure Typing Data includes a marker name 2200, reported peak data 2201, sample data 2202, and unit length data 2203. The reported peak data 2201 includes a list of fragment lengths of reported peaks. The sample data 2202 stores data in the form of a data structure Individual Data array. The data structure Individual Data includes a sample ID 2204 and peak data 2205 for each of i samples. The peak data 2205 stores data in the form of a list of a pair of a peak fragment length and a peak height.

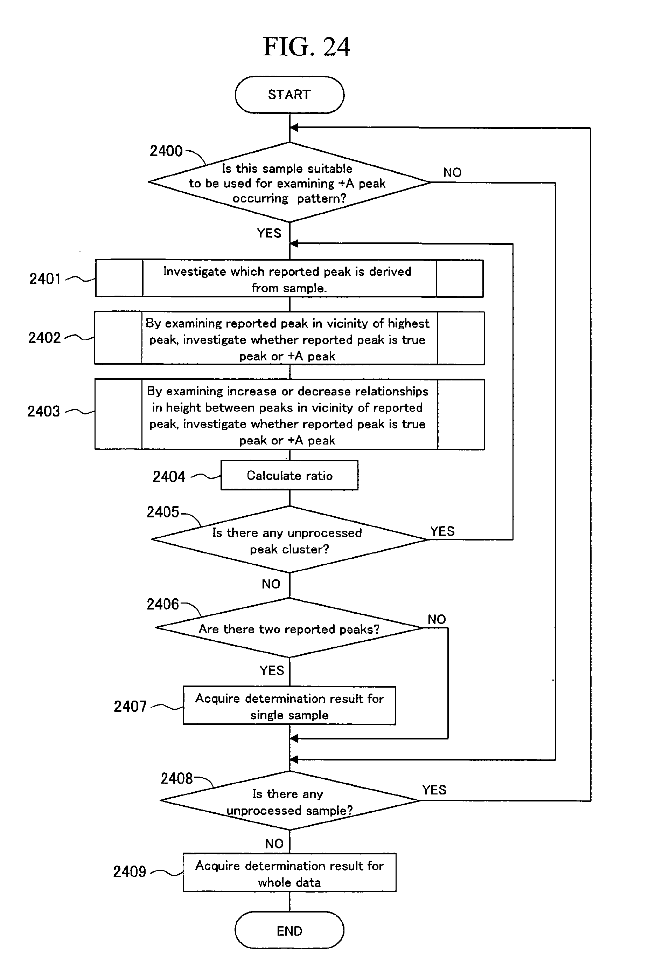

Next, processing performed in the gene information processing apparatus configured as above according to the present embodiment will be described in the following section. Fig. 23 is a flowchart which shows a schematic flow of processing in a gene information processing method. As described in Fig. 23, firstly, experimental data are loaded in the data structure Typing Data format from the experimental data DB 2100 (Step 2300). Next, a +A peak occurrence pattern is investigated based on the data of the reported peak data 2201 included in the Typing Data (Step 2301). This processing is performed by utilizing the sample selection part 2107, the reported peak selection part 2108, and the reported peak true/+A determination part 2109 which are included in the program memory 2105, and will be described further in the detailed flowchart shown in Fig. 24. Then, by referring to the +A peak occurrence pattern estimated in Step 2301, any noise peak included in the waveform is removed (Step 2302). This processing is described in detail in Matsumoto, T., et al.

The processing for estimating a +A peak occurrence pattern by use of the reported peak data 2201 in Fig. 23 will be explained further by referring to the detailed flowchart shown in Fig. 24. First, it is investigated whether or not a target sample is suitable to be used for examining a +A peak occurrence pattern by using the sample selection part 2107 (Step 2400). This processing can be completed by utilizing above-described Function 1-1. To be more precise, first, the highest peak is examined by referring to peak data 2205 of the target sample. Next, based on the reported peak data 2201, if no reported peak is located within one base pair from the highest peak, it is determined that the sample is not suitable to be used for estimating +A peak occurrence pattern. In addition, based on the unit length data 2203, if any reported peak is located in the vicinity of the highest peak with a distance that is not equivalent to the unit length, it is also determined that the sample is not suitable to be used for estimating +A peak occurrence pattern.

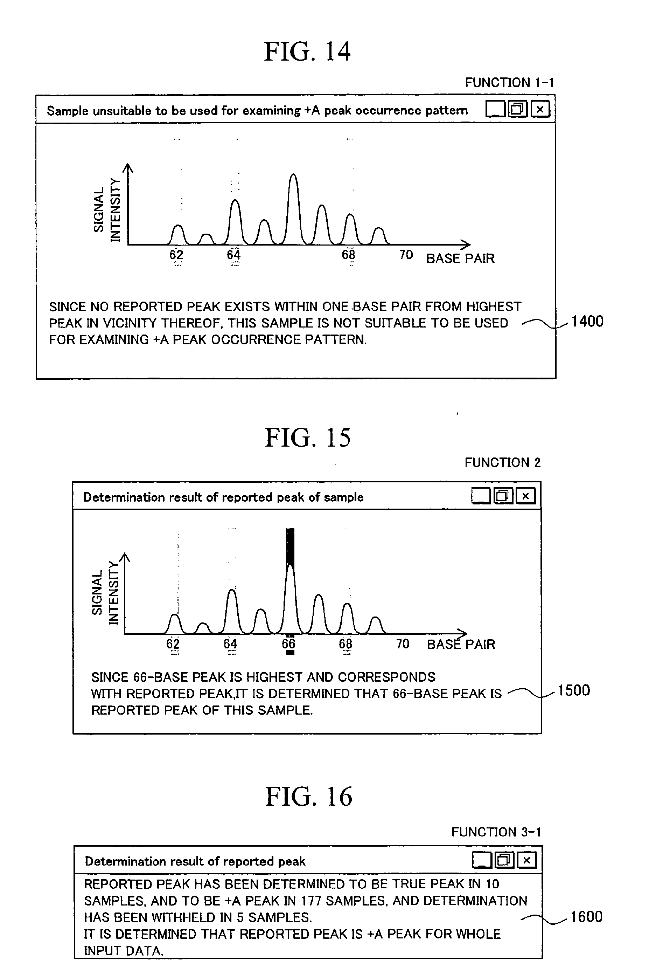

This determination result is displayed on a screen as shown in Fig. 14, for example. Alternatively, in place of screen display, other output methods, such as audio display and printer output, and recording methods, such as saving in a storage unit, such as a memory and a HDD, and in a storage medium, such as an optical disc, may be adopted. In other words, output methods are not limited.

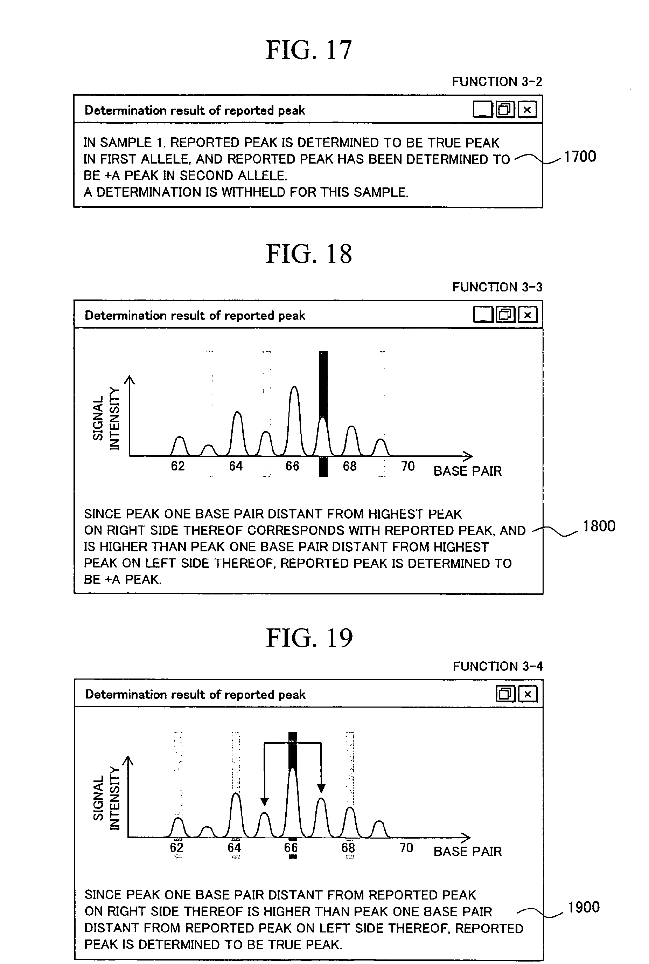

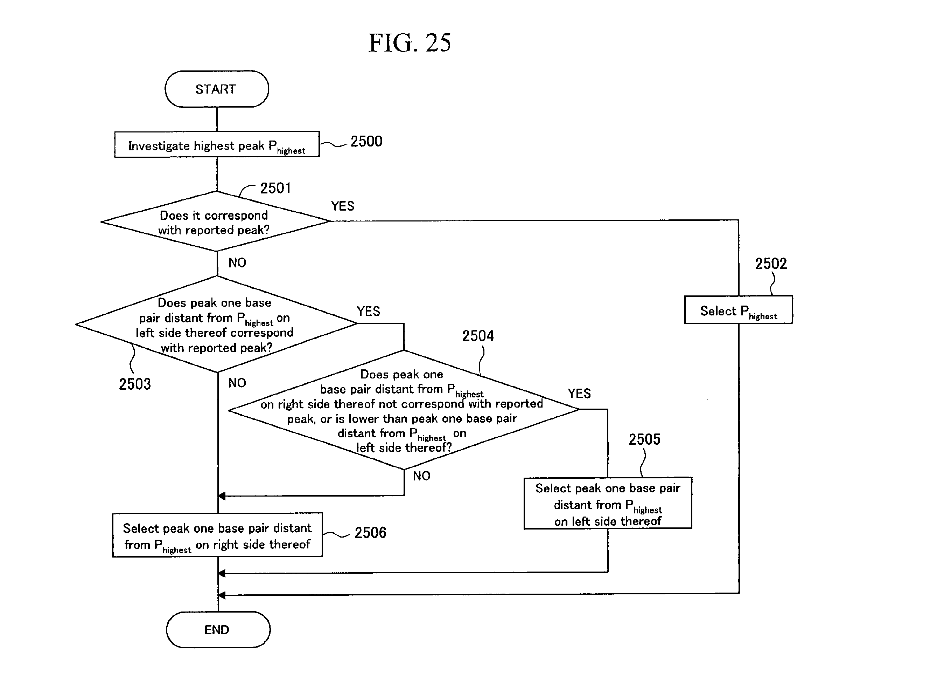

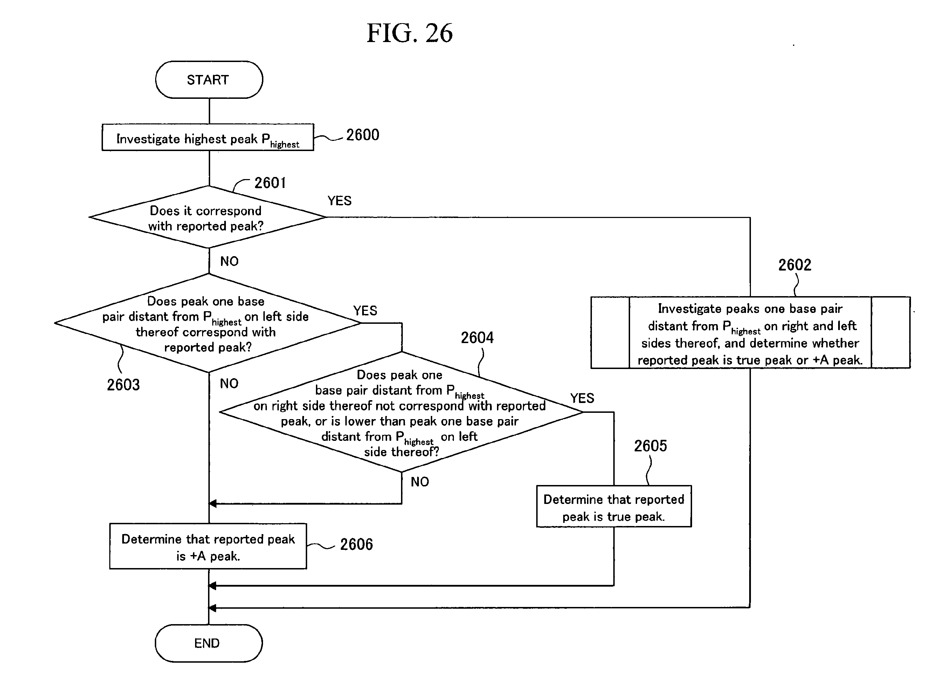

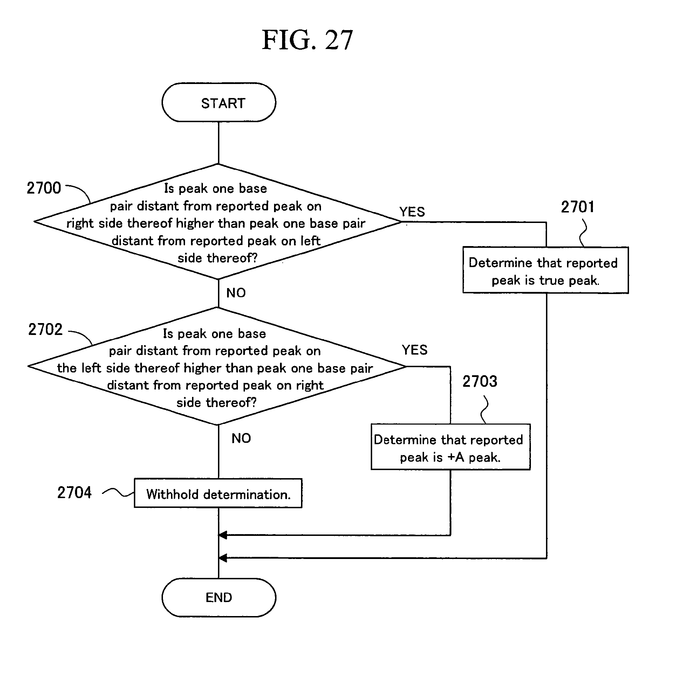

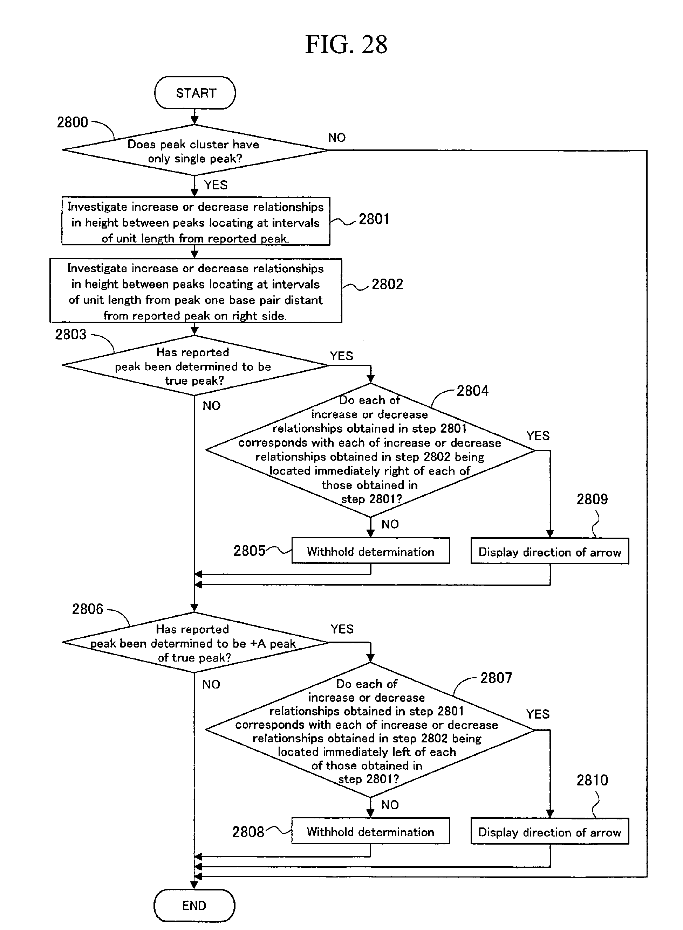

In the case where it has been determined that the sample is suitable in Step 2400, it is investigated which reported peak is derived from the sample by using the reported peak selection part 2108 (Step 2401). This processing is performed by utilizing above-described Function 2, and will be described further in the detailed flowchart shown in Fig. 25. The result of this processing is displayed on a screen as shown in Fig. 15. Then, the reported peak in the vicinity of the highest peak is examined by using the vicinity position confirmation processing section 2112 and the right-left comparison determination processing section 2113, and thereby, it is investigated whether the reported peak is a true peak or the +A peak thereof (Step 2402). This processing is performed by utilizing above-described Functions 3-3 and 3-4, and will be explained in detailed flowcharts shown in Figs. 26 and 27. This determination result is displayed on a screen as shown in Figs. 18 and 19. Then, increase or decrease relationships in height between the peaks in the vicinity of the reported peak are examined by using the increase or decrease relationship correspondence determination processing section 2114, and thereby, it is investigated whether the reported peak is a true peak or the +A peak thereof (Step 2403). This processing is performed by utilizing above-described Function 3-5, and will be described further in a detailed flowchart shown in Fig. 28. This determination result is displayed on a screen as shown in Fig. 20.

Based on these results, the ratio of heights between a true peak and the +A peak thereof is obtained (Step 2404). Next, if there is any unprocessed peak cluster, another series of processing is initiated from Step 2401 (Step 2405). If no unprocessed peak cluster exists, it is investigated whether or not the sample has two reported peaks (Step 2406). If so, with the allele majority decision processing section 2111, a determination result for the single sample is acquired (Step 2407). This processing can be performed by utilizing above-described Function 3-2. In other words, if determinations made for two reported peaks correspond with each other, the determination is adopted as a determination result for the single sample. If determinations do not correspond with each other, a determination for the sample is withheld. This determination result is displayed on a screen as shown in Fig. 17. Then, it is investigated whether or not there is any unprocessed sample (Step 2408). If so, another series of processing is initiated from Step 2400. If no, with the sample majority decision processing section 2110, a determination result for the whole data is acquired (Step 2409). This processing can be performed using above-described Function 3-1.

To be more precise, the number of samples in which the reported peak is a true peak and the number of samples in which the reported peak is the +A peak of the true peak are compared, and the determination made for the samples in the majority is adopted. This determination result is displayed on a screen as shown in Fig. 16.

Processing for investigating which reported peak is derived from a sample shown in Fig. 24 will be further described by referring to the detailed flowchart shown in Fig. 25. First, the highest peak P_{highest} is examined by referring to peak data 2205 of a target sample (Step 2500). Next, based on the reported peak data 2201, it is investigated whether or not P_{highest} corresponds with one of the reported peaks (Step 2501). If so (YES), P_{highest} is selected as the reported peak of the sample (Step 2502). If not (NO), it is then investigated whether or not the peak one base pair distant from P_{highest} on the left side thereof corresponds with one of the reported peaks (Step 2503). If so (YES), it is then investigated whether or not the peak one base pair distant from P_{highest} on the right side thereof does not correspond with any of the reported peaks, or whether or not the peak one base pair distant from P_{highest} on the right side thereof is lower than the peak one base pair distant from P_{highest} on the left side thereof (Step 2504). If either condition is fulfilled (YES), the peak one base pair distant from P_{highest} on the left side thereof is selected as the reported peak of the sample (Step 2505). If conditions in Steps 2503 and 2504 are not fulfilled (NO), the peak one base pair distant from P_{highest} on the right side thereof is selected as the reported peak of the sample (Step 2506).

Processing, shown in Fig. 24, for investigating whether the reported peak is the true peak or the +A peak thereof by investigating reported peaks in the vicinity of the highest peak will be further described by referring to the detailed flowchart shown in Fig. 26. First, the highest peak P_{highest} is examined by referring to peak data 2205 of a target sample (Step 2600). Next, based on the reported peak data 2201, it is investigated whether or not P_{highest} corresponds with one of the reported peaks (Step 2601). If so (YES), the peaks one base pair distant from P_{highest} on the right and left sides thereof are then investigated by using the right-left comparison determination processing section 2113, and thereby, it is determined whether the reported peak is the true peak or the +A peak thereof (Step 2602). This processing is performed by utilizing above-described Function 3-4, and will be further described in the detailed flowchart shown in Fig. 27. If not in Step 2601 (NO), it is then investigated whether or not the peak one base pair distant from P_{highest} on the left side thereof corresponds with one of the reported peaks (Step 2603). If so (YES), it is then investigated whether or not the peak one base pair distant from P_{highest} on the right side thereof does not correspond with any of the reported peaks, or whether or not the peak one base pair distant from P_{highest} on the right side thereof is lower than the peak one base pair distant from P_{highest} on the left side thereof (Step 2604). If either condition is fulfilled (YES), it is determined that the reported peak is the true peak (Step 2605). If any conditions in Steps 2603 and 2604 are not fulfilled (NO), it is determined that the reported peak is the +A peak (Step 2606).

Processing, shown in Fig. 26, for investigating whether the reported peak is the true peak of the +A peak thereof by investigating peaks one base pair distant from the highest peak on the right and left sides thereof will be further described by referring to the detailed flowchart shown in Fig. 27. First, it is investigated whether or not the peak one base pair distant from the reported peak on the right side thereof is higher than the peak one base pair distant from the reported peak on the left side thereof (Step 2700). If so, it is then determined that the reported peak is the true peak (Step 2701). If not, it is then investigated whether or not the peak one base pair distant from the reported peak on the left side thereof is higher than the peak one base pair distant from the reported peak on the right side thereof (Step 2702). If so, it is then determined that the reported peak is the +A peak (Step 2703). If not, in other words, if the height of the peak one base pair distant from the reported peak on the left side thereof is equal to that of the peak one base pair distant from the reported peak on the right side thereof, the determination is then withheld (Step 2704).

Processing, shown in Fig. 24, for investigating whether the reported peak is the true peak or the +A peak thereof by investigating increase or decrease relationship in height between peaks in the vicinity of the reported peak will be further described by referring to the detailed flowchart shown in Fig. 28. First, it is investigated whether or not a target sample has a unimodal cluster of peaks (Step 2800). If so (YES), for all pairs of peaks being located the unit length distant from each other on the right and left sides of the reported peak being as a center increase or decrease relationships in height are investigated (Step 2801). Next, for all pairs of peaks being located the unit length distant from each other on the right and left sides of the peak being both one base length distant from the reported peak on the right side thereof and as a center increase or decrease relationships in height are investigated(Step 2802). Then, it is investigated whether or not the reported peak has been determined to be the true peak in the flowcharts shown in Figs. 26 and 27 (Step 2803). If so (YES), it is investigated whether or not each of the increase or decrease relationships obtained in Step 2801 corresponds with each of the increase or decrease relationships obtained in Step 2802 being located immediately right of each of those obtained in Step 2801 (Step 2804). If so (YES), a direction of an arrow is indicated (Step 2809). If not (NO), a determination whether the reported peak is the true peak or the +A peak thereof is withheld (Step 2805). Then, it is investigated whether or not the reported peak has been determined to be the +A peak in the flowcharts shown in Figs. 26 and 27 (Step 2806). If so (YES), it is then investigated whether or not each of the increase or decrease relationships obtained in Step 2801 corresponds with each of the increase or decrease relationships obtained in Step 2802 being located immediately left of each of those obtained in Step 2801 (Step 2807). If so (YES), a direction of an arrow is indicated (Step 2810). If not (NO), a determination whether the reported peak is the true peak or the +A peak thereof is withheld (Step 2808).

Furthermore, in addition to the method for retrieving values stored in the database as described above, peaks which are included in a waveform used as input data may also be adopted as reported peak candidates. In this manner, a peak inappropriate to be a reported peak may also be selected as a candidate, and therefore, it is necessary to eliminate any inappropriate peak by user confirmation. However, even in such a case, the task to eliminate any peak inappropriate as a reported peak is significantly easier and simpler than that to precisely designate a true peak and the +A peak thereof for the whole input data. Hence, it is expected that user convenience can be largely improved by adopting the technique in the present embodiment.

As described above, by utilizing above-described functions, it becomes possible not only to obtain screen displays as shown in Figs. 14-20 and to estimate a +A peak occurrence pattern with a supplementary input of reported peak data, but also to display sample data which have served as the grounds for estimating the +A peak occurrence pattern. Fig. 14 shows a display example showing the reason for determining a sample to be unsuitable to be used for estimating a +A peak occurrence pattern by utilizing Function 1-1. As shown in the screen 1400, it has been determined whether or not a sample is suitable to be used for estimating a +A peak occurrence pattern by investigating a positional relationship of the highest peak with the neighboring reported peaks thereof. Fig. 15 shows a display example showing the reason for determining which reported peak is derived from a sample by utilizing Function 2. As shown in the screen 1500, it has been determined which reported peak is derived from a sample by investigating a positional relationship of the highest peak with the neighboring reported peaks thereof. Fig. 16 shows a display example showing the reason for determining whether a reported peak is a true peak or the +A peak thereof by utilizing Function 3-1. As shown in the screen 1600, the numbers of samples in which the reported peak has been determined to be the true peak, samples in which the reported peak has been determined to be the +A peak, and samples for which a determination has been withheld are indicated. Fig. 17 shows a display example showing the reason for determining whether a reported peak in a heterozygote is a true peak or the +A peak thereof by utilizing Function 3-2. As shown in the screen 1700, it is displayed whether or not determination results for the individual alleles correspond with each other. Fig. 18 shows a display example showing the reason for determining whether the reported peak is the true peak or the +A peak by utilizing Function 3-3. As shown in the screen 1800, it has been determined whether a reported peak is a true peak or the +A peak thereof by investigating a positional relationship of the highest peak with the neighboring reported peaks. Fig. 19 shows a display example showing the reason for determining whether a reported peak is a true peak or the +A peak thereof, when the highest peak corresponds with the reported peak, by utilizing Function 3-4. As shown in the screen 1900, it has been determined whether a reported peak is a true peak or the +A peak thereof by comparing the heights of the peaks one base pair distant from the true peak on the right and left sides thereof. Fig. 20 shows a display example showing the reason for determining whether a reported peak is a true peak or the +A peak thereof, when a target sample is a unimodal cluster of peaks, by utilizing Function 3-5. As shown in the screen 2000, it has been determined whether a reported peak is a true peak or the +A peak thereof by investigating increase or decrease relationships in height between peaks being located at intervals of a unit length.

As described above, according to the present embodiment, it is possible not only to accurately and rapidly estimate a +A peak occurrence pattern with a supplementary input of reported peak data but also to obtain sample data which have served as the grounds for estimating the +A peak occurrence and to output the results in a format which allows easier understanding.

Furthermore, the above-described functions in the present embodiment may be operated using a software program. If a software program is used, it is only necessary to provide a memory medium containing program code to a system or an apparatus, and then configure the computer (or CPU and MPU) of the system or the apparatus to be able to read the program code stored in the memory medium. In this case, the program code read from the memory medium becomes the one providing the above-described functions according to the embodiment; thus, the program code itself and the memory medium containing the program code are consequently to make up the system according to the present invention. Memory media which can be used for providing program code include a floppy ® disk, a CD-ROM, a DVD-ROM, a hard disk, an optical disk, a magneto optical disk, a CD-R, a magnetic tape, a nonvolatile memory card, and a ROM.

Furthermore, the OS (operating system) running on a computer, for example, may be configured to perform a part of, or the whole of, actual processing according to the instruction of a program code so that above-described functions in the embodiment can be provided by the processing. In addition, after program code read from a memory medium is written on a memory in a computer, the CPU of the computer may be configured to perform a part of or the whole actual processing according to the instruction of the program so that above-described functions in the embodiment can be provided by the processing.

Furthermore, with program code of the software that provides the functions of the embodiment being delivered via a network, and stored in a memory measures, such as a hard disk and a memory, or a memory medium, such as a CD-RW and a CD-R, in a system or an apparatus, the functions may also be provided by reading and executing the program code stored in such a memory means or a memory medium.

The present invention is applicable as an information processing apparatus for gene information.

## Claims

1. A gene information processing apparatus which is provided with a memory unit for storing, for each sample, an experimental result of an analysis of the length of a PCR amplification product of a DNA fragment as a set of the peak fragment length in terms of base pair and the peak height, and which performs discrimination of the peaks observed in the analysis result by use of the experimental analysis result stored in the memory unit as an input, the gene information processing apparatus comprising:
a processor which determines, on the basis of the data of the analysis result, whether or not each of the samples is suitable to be used for examining a +A peak occurrence pattern with a supplementary input of a list of peaks each being peaks of alleles possibly appearing for each microsatellite marker and being unable to be identified to be any one of a true peak and a +A peak with an additional single base A to the DNA fragment of the true peak (hereafter such peaks are referred to as "reported peaks"), and
an output controller performing output control of the result of the determination by the processor.

2. The gene information processing apparatus according to claim 1, wherein,
in a case where no reported peak is observed within one base pair from the highest peak, the processor determines that the highest peak is neither a real peak nor the +A peak thereof but an incidental noise peak, and determines that the sample is not suitable to be used for examining a +A peak occurrence pattern.

3. The gene information processing apparatus according to claim 1, wherein
unit length data are added to the supplementary input, and,
in the case where a reported peak is located at an interval different from the unit length of a microsatellite in the vicinity of the highest peak, the processor determines that the sample is not suitable to be used for examining a +A peak occurrence pattern.

4. The gene information processing apparatus according to any of claims 1 to 3, wherein, for a sample which has been determined to be suitable to be used for examining a +A peak occurrence pattern, the processor investigates a positional relationship of the highest peak and the neighboring reported peaks thereof, and identifies a reported peak of the sample.

5. The gene information processing apparatus according to claim 4, wherein, in the case where the highest peak corresponds with a reported peak, the processor determines that the highest peak is the reported peak of the sample.

6. The gene information processing apparatus according to claim 4, wherein, in the case where the peak one base pair distant from the highest peak on the left side thereof corresponds with a reported peak, and the peak one base pair distant from the highest peak on the right side thereof either does not correspond with a reported peak or is lower than the peak one base pair distant from the highest peak on the left thereof, the processor determines that the peak one base pair distant from the highest peak on the left side thereof is the reported peak of the sample.

7. The gene information processing apparatus according to any of claims 1 to 6, wherein the processor determines whether or not a true peak which should be observed is enumerated as a reported peak, and whether or not the +A peak of the true peak is enumerated as a reported peak.

8. The gene information processing apparatus according to claim 7, wherein the processor determines whether the reported peak is a true peak or the +A peak thereof for each sample, and makes a determination for the whole data by majority vote among samples.

9. The gene information processing apparatus according to claim 8, wherein the processor determines whether the reported peak in each of the two alleles in a heterozygote is a true peak or the +A peak thereof, and thereafter adopts the determination result when the determinations for both alleles agree with each other, and withholds a determination when the determinations do not agree with each other.

10. The gene information processing apparatus according to claim 9, wherein the processor investigates a positional relationship of the highest peak and the neighboring reported peaks thereof, and thereby determines whether the reported peak is a true peak or the +A peak thereof.

11. The gene information processing apparatus according to claim 10, wherein, in the case where the peak one base pair distant from the highest peak on the left side thereof corresponds with a reported peak, and the peak one base pair distant from the highest peak on the right side thereof either does not correspond with a reported peak or is lower than the peak one base pair distant from the highest peak on the left side thereof, the processor determines that the reported peak is a true peak.

12. The gene information processing apparatus according to claim 10, wherein, in the case where the peak one base pair distant from the highest peak on the right side thereof corresponds with a reported peak, and the peak one base pair distant from the highest peak on the left side thereof either does not correspond with a reported peak or is lower than the peak one base pair distant from the highest peak on the right side thereof, the processor determines that the reported peak is a +A peak.

13. The gene information processing apparatus according to any of claims 10 to 12, wherein, in the case where the highest peak corresponds with a reported peak, the processor determines whether the reported peak is the true peak or the +A peak thereof by comparing the heights of the peaks one base pair distant from the true peak on the right and left sides thereof.

14. The gene information processing apparatus according to claim 13, wherein, in the case where the peak one base pair distant from the highest peak on the right side thereof is higher than the peak one base pair distant from the highest peak on the left side thereof, the processor determines that the true peak is higher than the +A peak thereof, and that the reported peak and the true peak correspond with each other.

15. The gene information processing apparatus according to claim 13, wherein, in the case where the peak one base pair distant from the highest peak on the right side thereof is lower than the peak one base pair distant from the highest peak on the left side thereof, the processing device determines that the true peak is lower than the +A peak thereof, and the reported peak and the +A peak of the true peak correspond with each other.

16. The gene information processing apparatus according to claim 13, wherein, in the case where neither of the peaks one base pair distant from the highest peak on the right and left sides thereof is recognized, the processor withholds a determination whether the reported peak is the true peak or the +A peak thereof.

17. The gene information processing apparatus according to claim 9, wherein, in the case of a peak cluster with a single peak, the processor determines whether the reported peak is a true peak or the +A peak thereof by examining increase or decrease relationship in height between peaks being located at intervals of the unit length.

18. The gene information processing apparatus according to claim 17, wherein
a first increase or decrease relationship in height between peaks being located at intervals of the unit length from the reported peak and a second increase or decrease relationship in height between peaks being located at intervals of the unit length from the site one base pair distant from the reported peak on the right side thereof are investigated, and,
in the case where a plurality of pairs of each of the first increase or decrease relationships based on the starting point at a shorter base pair and each of the second increase or decrease relationships based on the starting point at a longer base pair have the first and second increase or decrease relationships corresponding with each other, the peaks having been investigated in terms of the increase or decrease relationship on the basis of the first increase or decrease relationships are determined to be true peaks, and the peaks having been investigated in terms of the increase or decrease relationship on the basis of the second increase or decrease relationships are determined to be +A peaks.

19. The gene information processing apparatus according to claim 17, wherein
a first increase or decrease relationship in height between peaks being located at intervals of a unit length from the reported peak and a second increase or decrease relationship in height between peaks being located at intervals of a unit length from a site one base pair distant from the reported peak on the left side thereof are investigated, and,
in the case where a plurality of pairs of each of the second increase or decrease relationships based on the starting point at a shorter base pair and each of the first increase or decrease relationships based on the starting point at a longer base pair have the first and second increase or decrease relationships corresponding with each other, the peaks having been investigated in terms of the increase or decrease relationship on the basis of the first increase or decrease relationships are determined to be +A peaks, and the peaks having been investigated in terms of the increase or decrease relationship on the basis of the second increase or decrease relationships are determined to be true peaks.

20. The gene information processing apparatus according to any of claims 1 to 19, further comprising a display device showing under control of the output controller the determination result.

21. A gene information processing method in which with an input of the result of analysis of the length of a PCR amplification product of a DNA fragment, a discrimination for peaks appearing in the analysis result is performed, the method comprising a step of determining whether or not a sample is suitable to be used for examining a +A peak occurrence pattern with a supplementary input of a list of peaks each being peaks of alleles possibly appearing for each microsatellite marker and being unable to be identified to be any one of a true peak and a +A peak with an additional single base A to the DNA fragment of the true peak (hereafter such peaks are referred to as "reported peaks").

22. A program causing a computer to perform the step described in claim 21.

23. A computer-readable storage media storing the program described in claim 22.
